(19) Europäisches Patentamt
European Patent Office
Office européen des brevets

(11) Veröffentlichungsnummer: **0 030 251**
**B1**

# (12) EUROPÄISCHE PATENTSCHRIFT

(45) Veröffentlichungstag der Patentschrift: **30.11.83**

(21) Anmeldenummer: **80103215.2**

(22) Anmeldetag: **10.06.80**

(51) Int. Cl.³: **C 07 C 59/68,** C 07 C 51/367, C 07 C 79/41, C 07 C 121/75 // A01N39/02

(54) **Verfahren zur Herstellung von (Phenoxi, bzw. Benzyl)- phenoxipropionsäuren und ihren Alkalisalzen.**

(30) Priorität: **11.12.79 DE 2949681**

(43) Veröffentlichungstag der Anmeldung: **17.06.81 Patentblatt 81/24**

(45) Bekanntmachung des Hinweises auf die Patenterteilung: **30.11.83 Patentblatt 83/48**

(84) Benannte Vertragsstaaten: **BE CH DE FR GB IT LI NL**

(56) Entgegenhaltungen:
**DE - A - 2 136 828**
**DE - A - 2 223 894**
**DE - A - 2 531 643**
**DE - A - 2 715 319**
**DE - A - 2 725 590**
**GB - A - 1 422 679**
**US - A - 3 113 965**
**US - A - 3 493 604**

(73) Patentinhaber: **HOECHST AKTIENGESELLSCHAFT, Postfach 80 03 20, D-6230 Frankfurt am Main 80 (DE)**

(72) Erfinder: **Koch, Manfred, Dr., Kreuzheck 2, D-6239 Eppstein/Taunus (DE)**
Erfinder: **Herbrechtsmeier, Peter, Dr., Berliner Strasse 10, D-6238 Hofheim am Taunus (DE)**

**EP 0 030 251 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur Herstellung von (Phenoxy, bzw. Benzyl)-phenoxypropionsäuren und ihren Alkalisalzen

Die vorliegende Erfindung betrifft ein Verfahren zur Herstellung von (Phenoxy- bzw. Benzyl)phenoxypropionsäuren der Formel

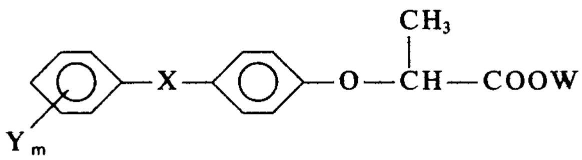

(I)

worin

Y ein Rest aus der Gruppe Halogen, vorzugsweise Chlor oder Brom; $CF_3$, $NO_2$ oder CN,
X Sauerstoff oder $-CH_2-$,
W Wasserstoff, Natrium oder Kalium bedeuten und
m 1 oder 2 ist,

sowie von deren optisch aktiven D-Isomeren.

Es ist bekannt, daß alkyl- und/oder halogen-substituierte Phenoxyessig-, bzw. -propionsäuren, welche als sogenannte Wuchsstoffherbizide (im folgenden Wuchsstoffsäuren genannt) von großer wirtschaftlicher Bedeutung sind, durch Umsetzung von Alkaliphenolaten mit den Alkalisalzen der entsprechenden 2-Halogenalkancarbonsäuren hergestellt werden können.

Im allgemeinen wird die Umsetzung so durchgeführt, daß Alkaliphenolat und das Alkalisalz der 2-Halogenalkancarbonsäure im Molverhältnis 1 : 1 in Wasser als Lösungs-, bzw. Verdünnungsmittel kondensiert werden (siehe G. Erfurt et al., Chem. Technik 15 [1964] Nr. 4, Seite 199; DD-PS 64 279, 64 972, 64 723).

Aufgrund der dabei nicht vermeidbaren Nebenreaktion (der Hydrlolyse von Halogenalkancarbonsäuresalz zu Hydroxyalkancarbonsäuresalz) ist die Ausbeute nicht befriedigend (70—85% d. Th.). Auch wird die Ausbeute beim Übergang von 2-Chloressigsäure zu höheren 2-Chloralkancarbonsäuren wie 2-Chlorpropionsäure und 2-Chlorbuttersäure erheblich vermindert (siehe DE-PS 1 153 762).

Die Ausbeute an Wuchsstoffsäuren kann durch Zurückdrängung der Hydrolyse erhöht werden. Bekanntgeworden sind folgende Möglichkeiten:

a) Verwendung von wasserfreien Reaktanten (US-PS 2 651 659). Obwohl hier wesentlich bessere Ausbeuten an Wuchsstoffsäuren erzielt werden, ist diese Variante für eine technische Durchführung wenig geeignet, da
   - die Herstellung von wasserfreien Agentien, insbesondere der hydrolyseempfindlichen Alkalisalze der 2-Halogenalkancarbonsäuren schwierig und teuer ist,
   - große Volumina organischer Lösungsmittel notwendig sind,
   - Feststoffe oder Suspensionen zudosiert werden müssen,
   - als unerwünschte Nebenreaktion in größerem Umfang Lactate (durch Alkylierung der Phenoxyalkancarbonsäuresalze mit Halogenalkancarbonsäuresalz) entstehen.

b) Teilweiser Ersatz von Wasser als Lösungs- und Verdünnungsmittel durch höhersiedende Alkohole (DE-PS 1 153 762, US-PS 2 914 558, SU-PS 187 766, Jap. Anm. Nr. 48 705/65), durch Kohlenwasserstoffe (US-PS 2 480 817) oder durch überschüssiges freies Phenol (US-PS 4 035 496, DD-PS 50 622).

c) Anwendung eines Überschusses bis zu 100% an Alkaliphenolat bezogen auf eingesetztes 2-Halogenalkancarbonsäuresalz zur Erhöhung der Geschwindigkeit der Hauptreaktion gegenüber der Hydrolyse-Nebenreaktion (US-PS 4 035 416, DE-PS 1 153 762, JA-AS 74/24 463, US-PS 3 257 453).

d) Reduzierung der im Reaktionsgemisch vorhandenen Wassermenge durch Abdestillierung von Wasser während der Umsetzung. Bei der in US-PS 4 035 416 beschriebenen Versuchsanordnung wird Natronlauge mit überschüssigem 2,4-Dichlorphenol versetzt und ein Teil (ca. 35%) des im Reaktionsgemisch vorhandenen Wassers abdestilliert, anschließend werden äquimolare Mengen an Natronlauge und Chloressigsäure, im Unterschuß bezogen auf Natriumdichlorphenolat, simultan zudosiert, wobei gleichzeitig Wasser abdestilliert wird.

Grundsätzlich gemeinsam ist allen bekannten Verfahren zur Herstellung von Wuchsstoffsäuren:

- Die zur Umsetzung vorgesehene Phenolmenge wird als Alkaliphenolat vorgelegt, dazu wird 2-Halogenalkancarbonsäuresalz in einer Portion, in mehreren Portionen oder kontinuierlich zugesetzt. In manchen Fällen wird die insgesamt notwendige Alkalimenge als überschüssiges

Alkaliphenolat oder als Mischung von Alkaliphenolat und Alkalihydroxid vorgelegt und 2-Halogenalkancarbonsäure zudosiert.

— Die Phenolkomponente wird nicht quantitativ umgesetzt. Nach erfolgter Umsetzung ist eine Abtrennung und Rückgewinnung von nichtumgesetztem Phenol notwendig.

Eine analoge Herstellung von (Phenoxy- bzw. Benzyl)phenoxypropionsäuren der Formel I ist nach oben genannten Verfahren nicht möglich, da es nur durch aufwendige und langwierige Reinigungsoperationen unter Verlust gelingt, die als Ausgangsmaterialien verwendeten Phenoxy- bzw. Benzylphenole von den Verbindungen der Formel I abzutrennen.

Zwar sind für die Herstellung dieser Verbindungen ebenfalls bereits eine Anzahl von Verfahren bekanntgeworden, die jedoch gleichfalls nicht zu quantitativen Ausbeuten führen:

In der DE-OS 1 668 896 wird neben anderen Herstellungsmethoden die Umsetzung von α-Halogenfettsäuren mit Phenoxyphenolen in wäßriger, stark alkalischer Lösung beschrieben. Verfährt man nach dieser, an den frühen Stand der Wuchsstoffsäurenherstellung angelehnten Verfahrensweise, indem man Phenoxyphenole mit einem Überschuß von 20% 2-Chlorpropionsäure in wäßriger stark alkalischer Lösung kondensiert, erhält man nur einen Umsatz von 82% d. Th. Ein Umsetzungsgrad >95% der Phenolkomponente wird erst bei wesentlich höherem Überschuß an 2-Chlorpropionsäure (>40%) erreicht. Ein solches Verfahren ist zur quantitativen Umsetzung der Phenoxyphenole in technischem Maßstab nicht geeignet.

Die Verbesserung dieser Umsetzung durch Ersatz von Wasser als Lösungs- bzw. Verdünnungsmittel durch einen Kohlenwasserstoff wie Toluol oder Xylol und azeotrope Abdestillierung von Wasser während der Umsetzung gemäß Abschnitt b) und d) weiter oben führt ebenfalls nicht zu einer quantitativen Umsetzung der Phenole. So wird mit einem Überschuß von 20% der 2-Chlorpropionsäure (bezogen auf eingesetztes Phenol) nur ein Umsatz von 92—93% der Phenolkomponente erzielt.

Besondere Schwierigkeiten bereitet die Herstellung von optisch aktiven D-Isomeren der Verbindungen der Formel I. Die üblicherweise angewandte Racemattrennungsmethode, ausgehend von einer optisch inaktiven 2-Phenoxypropionsäure unter Verwendung einer optisch aktiven Hilfsbase, ist in technischem Umfang nicht durchführbar. Die bei diesem Verfahren erforderliche fraktionierende Kristallisation ist arbeitsaufwendig und verlustreich. Weiterhin entsteht in stöchiometrisch gleicher Menge das unerwünschte L-Enantiomere, das nur in seltenen Fällen wieder in erneut spaltbares Racemat überführt werden kann.

Die in der DE-OS 2 758 002 beschriebene Methode führt ausgehend von 2-Methan-sulfonyloxy- oder Toluolsulfonyloxypropionsäure-Derivaten zu Derivaten der gewünschten 2-Phenoxypropionsäuren, aus denen dann im allgemeinen durch alkalische oder saure Verseifung die Methansulfonyl- oder Toluolsulfonylgruppe wieder abgespalten werden muß. Bei diesem Reaktionsschritt besteht in hohem Maße das Risiko einer Racemisierung des hergestellten Enantiomeren.

Entsprechendes gilt für die Umsetzung geeignet substituierter Phenole mit optisch aktiven 2-Brompropionsäurederivaten in organischen Lösungsmitteln wie Aceton, Methylethylketon oder Acetonitril in Gegenwart einer Base (H. J. Nestler und H. Bieringer, Zeitschr. für Naturforsch. 1980, im Druck).

Die optisch aktiven 2-Sulfonyloxy-propionsäure-Derivate wie auch die optisch aktiven 2-Brompropionsäure-Derivate sind darüber hinaus nur in beschränktem Maße verfügbar. Als gut zugängliche optisch aktive Ausgangsprodukte stehen hingegen die L-2-Chlorpropionsäurealkylester, insbesondere der Methylester, in technischem Maßstab zur Verfügung.

Die Umsetzung der 2-Chlorpropionsäure-methylester mit den in Frage kommenden Phenolen gelingt in organischen Lösungsmitteln wie z. B. Aceton, Methylethylketon oder Acetonitril in Gegenwart organischer oder anorganischer Basen aber nur unvollständig und ist damit für die praktische Anwendung nicht geeignet.

Eine fast quantitative chemische Umsetzung ist zwar zu erreichen, wenn man in einem höhersiedenden organischen Lösungsmittel wie z. B. Toluol, Xylol oder Chlorbenzol ein Alkalisalz des Phenols mit einem 2-Chlorpropionsäureester umsetzt und anschließend zur Säure verseift; jedoch tritt im Falle der optisch aktiven 2-Chlorpropionsäureester bei dieser Verfahrensweise nahezu vollständige Racemisierung ein.

Zwar bleibt die optische Aktivität weitgehend erhalten, wenn man an Stelle des Esters ein Alkalisalz der optisch aktiven 2-Chlorpropionsäuren für diese Reaktion verwendet. Die Herstellung dieser Salze durch Verseifung der technisch allein zugänglichen L-2-Chlorpropionsäureester ist jedoch wegen der Racemisierungsmöglichkeit ebenfalls problematisch. Außerdem tritt als Konkurrenzreaktion zur Esterverseifung eine Verseifung des α-ständigen Chloratoms unter Bildung von Milchsäure ein.

In der deutschen Patentschrift 1 543 841 wird ein Verfahren zur Herstellung von D-Phenoxypropionsäuren beschrieben, bei welchem Alkalisalze der L-2-Chlorpropionsäure in situ erzeugt und mit Phenolaten umgesetzt werden, wodurch die Racemisierung verhindert wird. Bei diesem Verfahren ist es jedoch erforderlich, enge Temperaturgrenzen zwischen 5 und 35°C zu beachten. Wegen der Reaktivität der Chlorpropionsäureester einerseits und der Kristallisationsneigung der entstehenden Salze andererseits sind diese Grenzen aber schon bei geringfügig vergrößerten Reaktionsansätzen praktisch nicht mehr einzuhalten. Vielmehr besteht hierbei die Gefahr einer unkontrollierbar

ablaufenden Spontanreaktion mit Auftreten starker Überhitzungen. Das bei dem Verfahren nach DE-PS 1 543 841 im Reaktionsgemisch verbleibende Wasser verhindert außerdem die vollständige Umsetzung der Reaktanden, so daß stets beträchtliche Phenolgehalte in den Endprodukten in Kauf genommen werden müssen.

Mit dem erfindungsgemäßen Verfahren gelingt es überraschenderweise, die Nachteile der beschriebenen Verfahren zu vermeiden und Verbindungen der Formel I in Ausbeuten von über 99% sowie deren D-Isomeren in Ausbeuten von über 95% und in hoher optischer Reinheit zu erhalten.

Das Verfahren ist dadurch gekennzeichnet, daß man zu einer Lösung eines Phenols der Formel II

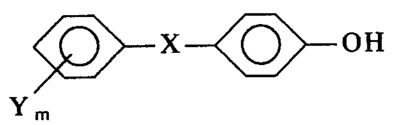

und einer äquimolaren oder geringfügig überschüssigen Menge 2-Chlorpropionsäure oder eines 2-Chlor-propionsäureniederalkylesters der Formel III

$$Cl-\underset{|}{\overset{CH_3}{CH}}-COOR \qquad (III)$$

worin R Wasserstoff oder $(C_1-C_4)$Alkyl bedeutet, in einem mit Wasser Azeotrope bildenden organischen Lösungsmittel in der Siedehitze eine doppelt molare Menge oder einen geringen Überschuß (bezogen auf III) eines wäßrigen Alkalihydroxids zugibt, von Beginn der Zugabe an das zugeführte bzw. bei der Reaktion entstehende Wasser und gegebenenfalls entstandes Lösungsmittel azeotrop abdestilliert und gewünschtenfalls aus den erhaltenen Alkalisalzen der Formel I durch Ansäuern die freien Säuren herstellt.

Der zur quantitativen Umsetzung (>99,5%) der Phenoxy-, bzw. Benzylphenole der allgemeinen Formel II notwendige Überschuß an Verbindung III beträgt mindestens 10% (bezogen auf eingesetztes Phenol), im allgemeinen wird ein Überschuß von 12—20% angewandt.

Die Umsetzung wird im allgemeinen bei Temperaturen von 80—130, vorzugsweise 90—120° durchgeführt.

Das Verfahren wird im folgenden näher beschrieben:

In einem mit Wasserabscheider ausgerüsteten Reaktionsgefäß werden das umzusetzende Phenol und 2-Chlorpropionsäure oder -propionsäureester in einem mit Wasser Azeotrope bildenden organischen Lösungsmittel, wie z. B. Toluol, Xylol, Chlorbenzol oder Dichlorbenzol gelöst. Zu dieser Lösung wird unter gleichzeitigem Abdestillieren von Wasser unter vermindertem Druck eine 20—60%ige, vorzugsweise 30—50%ige wäßrige Alkalihydroxydlösung zudosiert.

Je nach Wahl des Lösungsmittels wird die Reaktion bei Normaldruck oder vermindertem Druck durchgeführt.

Der gegebenenfalls anzuwendende Unterdruck hängt außerdem von der Reaktionstemperatur ab. Als zweckmäßig hat es sich erwiesen, die Reaktion nicht unterhalb 80° durchzuführen, da sonst die Reaktionsgeschwindigkeit zu langsam wird. Dies hat zur Folge, daß Drucke von 200 Millibar im allgemeinen nicht unterschritten werden. Andererseits muß für einen raschen Abtransport des zugeführten bzw. gebildeten Wassers gesorgt werden. Bei Verwendung von Xylol als Lösungsmittel und einer Reaktionstemperatur von 110° beträgt der Druck ca. 500 mbar.

Bei der Herstellung von optisch aktiven D-Isomeren der Formel I geht man von L-2-Chlorpropionsäure oder L-2-Chlorpropionsäureester als Ausgangsstoff aus.

Pro Mol Phenol werden ca. 1,1—1,4 Mol, bevorzugt 1,1—1,2 Mol Chlorpropionsäure(ester), vorzugsweise Chlorpropionsäuremethylester, 2,2—2,8 Mol, und vorzugsweise 2,2—2,5 Mol Alkalihydroxyd verwendet.

Als Alkalihydroxyde kommen in erster Linie NaOH und KOH in Frage. Die Zulaufgeschwindigkeit wird so geregelt, daß sich Wasserzufuhr zuzüglich Reaktionswasserbildung und Wasserabscheidung in etwa entsprechen. Zusammen mit dem Wasser destilliert ggf. der durch Verseifung des Esters entstandene Alkohol, im bevorzugten Fall Methanol, ab.

Nach beendeter Zugabe der Alkalihydroxydlösung wird ca. 15 Minuten bei gleichbleibender Reaktionstemperatur nachgerührt. Die entstandenen Alkalisalze der Formel I können direkt in Derivate der Säure (z. B. Ester) oder durch Ansäuern in die freie Säure überführt werden.

Die nach dem erfindungsgemäßen Verfahren erhaltenen Verbindungen der Formel I sind hochwirksame Gräserherbizide oder Vorprodukte für andere Säurederivate, wie zum Beispiel Ester, welche ebenfalls hochwirksam selektive Gräserherbizide darstellen (DE-OS 2 223 894, 2 433 967, 2 601 548, 2 417 487 und 2 758 002).

Die Ausbeuten betragen über 98%, in vielen Fällen über 99,5%. Die optischen Reinheiten liegen nicht mehr als maximal 25%-Punkte unter denen der eingesetzten optisch aktiven 2-Chlorpropionsäureester. Die optische Reinheit in % ergibt sich rechnerisch wie folgt: %-Gehalt D-Form minus

%-Gehalt L-Form = optische Reinheit.

Von dem beschriebenen Stand der Technik unterscheidet sich das erfindungsgemäße Verfahren dadurch, daß das Phenoxy- bzw. Benzylphenol der allgemeinen Formel II als freies Phenol eingesetzt wird. Bei der Zudosierung von Natronlauge, wird nur so viel Phenolat gebildet, wie mit 2-Chlorpropionat abreagieren kann. Damit wird ein Überschuß von Alkali im Reaktionsgemisch verhindert, wodurch eine wesentliche Verminderung des durch Hydrolyse verursachten Verlusts an Verbindung III erreicht wird.

## Beispiele

### Beispiel 1

2-[4'-(2'',4''-Dichlorphenoxy)-phenoxy]-propionsäure

Zu einer Lösung von 255 Gew.-Teilen 4-(2',4'-Dichlorphenoxy)-phenol und 132 Gew.-Teilen 2-Chlorpropionsäure in 1200 Gew.-Teilen Xylol werden 208 Gew.-Teile 50%ige Natronlauge unter einem Vakuum von 270 mb bei etwa 90°C zudosiert. Die Reaktionstemperatur wird durch Abdestillieren von im Reaktionsgemisch befindlichem Wasser bei 90°C gehalten. Nach beendeter Zugabe wird 15 min bei 90°C nachgerührt, anschließend belüftet. Die Mischung wird mit 400 Gew.-Teilen Wasser versetzt und 10 min bei 85°C gerührt. Anschließend säuert man bei 85°C mit 120 Teilen Phosphorsäure an und rührt 10 min nach. Nach Abtrennung der Wasserphase im Scheidetrichter wird Xylol unter 80 mb abdestilliert und der Feststoff bei 60°C, 250 mb getrocknet. Man erhält 327 Gew.-Teile 2-[4'-(2'',4''-Dichlorphenoxy)-phenoxy]-propionsäure. Der Gehalt an reinem Endprodukt beträgt 99,5%, entsprechend einer Ausbeute von 99,5% d. Th. Der Restgehalt an 4-(2'',4''-Dichlorphenoxy)-phenol beträgt 0,3%.

### Beispiel 2

2-[4'-(2'',4''-Dichlorphenoxy)-phenoxy]-propionsäure

127,5 Gew.-Teilen 4-(2',4'-Dichlorphenoxy)-phenol und 69,2 Gew.-Teilen 2-Chlorpropionsäuremethylester werden in 650 Gew.-Teilen Xylol vorgelegt. Zu dieser Lösung tropft man bei etwa 110°C und einem Vakuum von 500 mb 100 Gew.-Teile 50%ige Natronlauge zu, wobei gleichzeitig Wasser und Methanol am Wasserabscheider azeotrop abdestilliert wird. Nach beendeter Zugabe wird 15 min bei 110°C nachgerührt, anschließend wird die Reaktionsapparatur belüftet. Die Aufarbeitung erfolgt wie in Beispiel 1 beschrieben. Man erhält 163,3 Gew.-Teile 2-[4'-(2'',4''-Dichlorphenoxy)-phenoxy]-propionsäure. Der Gehalt an reinem Endprodukt beträgt 99,8%, entsprechend 99,6% d. Th. Der Restgehalt an 4-(2',4''-Dichlorphenoxy)-phenol beträgt 0,15%.

### Beispiel 3

D-2-[4-(2,4-Dichlorphenoxy)-phenoxy]-propionsäure

Zu einer Lösung von 102 g (0,4 Mol) 4-(2,4-Dichlorphenoxy)-phenol und 56,35 g (0,46 Mol) L-2-Chlorpropionsäuremethylester in 450 ml Xylol gibt man bei 110°C, 500 mb unter kräftigem Rühren 77,7 g 51,5%ige Natronlauge (1,0 Mol) im Verlauf von etwa einer Stunde zu. Über einen Wasserabscheider wird von Beginn der Zugabe an das Gemisch von entstandenem Methanol und zugeführtem bzw. gebildetem Wasser azeotrop entfernt.

Nach Vollendung der Zugabe wird 15 Minuten bei 110°C, 500 mb nachgerührt, auf Normaldruck entspannt und bei 90°C zunächst 100 ml Wasser zugegeben, anschließend mit verdünnter Schwefelsäure die D-2-[4-(2,4-Dichlor-phenoxy)-phenoxy]-propionsäure in Freiheit gesetzt. Nach Abtrennen der Wasserphase und Abdestillieren von Xylol unter vermindertem Druck erhält man 129,8 g (99% d. Th.) D-2-[4-(2,4-Dichlorphenoxy)-phenoxy]-propionsäure mit einer Reinheit nach Gaschromatographie von 98,8%.

Der Restgehalt an 4-(2,4-Dichlorphenoxy)-phenol beträgt 0,3%.

Die optische Reinheit wird zweckmäßig nach Überführung in den Methylester ermittelt. Der optische Drehwert des nach obiger Umsetzung erhaltenen Produkts beträgt $[\alpha]_D^{22} = 21,14$ (1 molar in Chloroform), entsprechend einer optischen Reinheit von 76%.

In gleicher Weise erhält man bei Verwendung eines L-Chlor-propionsäureesters mit einer optischen Reinheit von 95% (entsprechend 97,5% L-Form) die folgenden Verbindungen mit optischen Reinheiten von über 70% (entsprechend 85% D-Form):

D-2-[4-(4-Chlorphenoxy)-phenoxy]-propionsäure
D-2-[4-(2,4-Dichlorphenoxy)-phenoxy]-propionsäure
D-2-[4-(4-Trifluormethylphenoxy)-phenoxy]-propionsäure
D-2-[4-(2,4-Dichlorbenzyl)-phenoxy]-propionsäure
D-2-[2-Chlor-4-trifluormethylphenoxy)-phenoxy]-propionsäure
D-2-[4-(4-Brom-2-chlorphenoxy)-phenoxy]-propionsäure sowie deren Na- und K-Salze

**Patentansprüche**

1. Verfahren zur Herstellung von (Phenoxy- bzw. Benzyl)-phenoxypropionsäuren und ihren Alkalisalzen der allgemeinen Formel I,

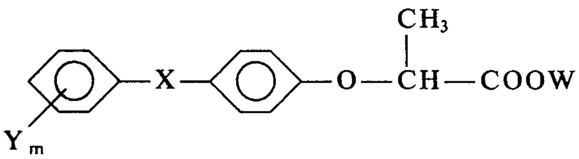
(I)

in der

Y ein Rest aus der Gruppe Halogen, vorzugsweise Chlor oder Brom; $CF_3$, $NO_2$ oder CN
X Sauerstoff oder $-CH_2-$
W Wasserstoff, Natrium oder Kalium bedeuten und
m 1 oder 2 ist,

sowie von deren optisch aktiven D-Isomeren, durch Umsetzung von Phenolen der Formel II

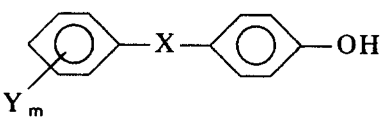
(II)

mit 2-Chlorpropionsäure oder einem 2-Chlorpropionsäureniederalkylester bzw. deren optisch aktiven L-Isomeren in Gegenwart von Alkalihydroxid, dadurch gekennzeichnet, daß man zu einer Lösung eines Phenols der Formel II und einer äquimolaren oder geringfügig überschüssigen Menge der 2-Chlorpropionsäure oder des 2-Chlorpropionsäureesters in einem mit Wasser Azeotrope bildenden organischen Lösungsmittel in der Siedehitze eine doppelt molare Menge (bezogen auf III) oder einen geringen Überschuß eines wäßrigen Alkalihydroxids zugibt, von Beginn der Zugabe an das zugeführte bzw. bei der Reaktion entstehende Wasser und gegebenenfalls entstandenes Lösungsmittel azeotrop abdestilliert und gewünschtenfalls aus den erhaltenen Alkalisalzen der Formel I durch Ansäuern die freien Säuren herstellt.

2. Verfahren gemäß Anspruch 1, dadurch gekennzeichnet, daß man bei Temperaturen von 80—130°, vorzugsweise 90—120° und Drucken zwischen 200 mbar und Normaldruck arbeitet.

**Claims**

1. A process for the preparation of (phenoxy- or benzyl-)-phenoxypropionic acids and the alkali metal salts thereof corresponding to the general formula I

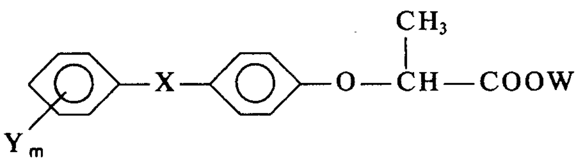
(I)

in which

Y is a radical from the group of halogen, preferably chlorine or bromine; $CF_3$, $NO_2$ or CN,
X is oxygen or $-CH_2-$,
W is hydrogen, sodium or potassium, and
m is 1 or 2,

and the optically active D-isomers thereof, by reacting phenols of the formula II

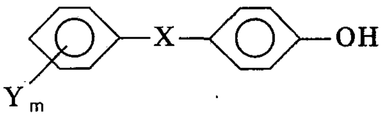

(II)

with 2-chloropropionic acid or 2-chloropropionic acid lower alkyl esters or optically active L-isomers thereof in the presence of alkali hydroxides, which comprises adding to a solution of the phenol of formula II and an equimolar amount or a slight excess of the 2-chloropropionic acid or 2-chloropropionic acid ester in an organic solvent capable of forming an azeotropic mixture with water, an about double molar amount (relative to III) or a slight excess thereover of an aqueous alkali hydroxide at reflux temperature while distilling off azeotropically the water introduced or formed during the reaction and if necessary, solvent formed in the reaction and optionally converting the alkali salts of the formula I obtained to the free acids by acidification.

2. The process as claimed in Claim 1, which comprises operating at temperatures of from 80 to 130° C, preferably 90—120° C, and under a pressure between 200 mbar and normal.

**Revendications**

1. Procédé de préparation d'acides (phénoxy ou benzyl)-phénoxy-propioniques et de leurs sels de métaux alcalins, qui répondent à la formule générale (I):

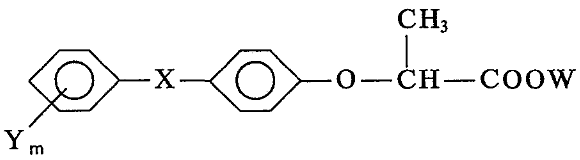

(I)

dans laquelle:

Y représente un radical pris dans l'ensemble constitué par les halogènes, de préférence le chlore et le brome, et les radicaux $CF_3$, $NO_2$ et CN,
X représente l'oxygène ou $-CH_2-$,
W représente l'hydrogène, le sodium ou le potassium et
m est égal à 1 ou à 2,

ainsi que de leurs isomères D optiquement actifs, par réaction de phénols de formule (II):

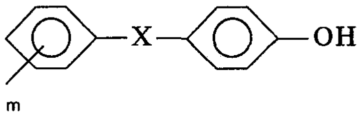

(II)

avec l'acide chloro-2 propionique ou un chloro-2 propionate d'alkyle inférieur (III) ou leurs isomères L optiquement actifs, en présence d'un hydroxyde de métal alcalin, procédé caractérisé en ce qu'à une solution d'un phénol de formule (II) et d'une quantité équimolaire, ou légèrement supérieure, de l'acide chloro-2 propionique ou de l'ester de l'acide chloro-2 propionique dans un solvant organique formant un azéotrope avec l'eau, on ajoute, à la température d'ébullition, une quantité molaire double (par rapport au composé (III)) ou un léger excès d'un hydroxyde de métal alcalin aqueux, on chasse par distillation azéotropique, dès le début de l'addition, l'eau introduite ou celle qui se forme au cours de la réaction et le solvant éventuellement formé, et, si on le désire, on prépare à partir des sels de métaux alcalins de formule (I) obtenus, par acidification, les acides libres.

2. Procédé selon la revendication 1 caractérisé en ce qu'on opère à des températures de 80 à 130° C, de préférence de 90 à 120° C, et sous des pressions comprises entre 200 mbar et la pression normale.